# EUROPEAN PATENT SPECIFICATION

(11) **EP 2 638 876 B1**
(45) Date of publication and mention of the grant of the patent: **07.12.2016**
(21) Application number: 13171457.8
(22) Date of filing: 12.08.2005
(51) Int. Cl.: A61B 18/22, A61F 9/008, A61C 1/00

(54) **Laser handpiece architecture and methods**
Laserhandstückarchitektur und Verfahren
Procédés et architecture de pièce à main laser

(30) Priority: 13.08.2004 US 601416 P; 18.09.2004 US 610760 P
(43) Date of publication of application: 18.09.2013
(62) Divisional of application: 05814100.3
(73) Proprietor: Biolase, Inc., Irvine, CA 92618 (US)
(72) Inventor: Jones, Jeffrey W., Robertson, WY 82944 (US); Boutoussov, Dmitri, Dana Point, CA 92629 (US)
(74) Representative: Kopf Westenberger Wachenhausen Patentanwälte PartG mbB

(56) References cited:
- WO-A1-98/41355
- WO-A2-2005/070129
- WO-A2-2006/012461
- DE-A1- 3 911 853
- DE-A1- 4 401 989
- US-A- 5 304 173
- US-A- 5 435 724
- US-B1- 6 389 193

## Description

### BACKGROUND OF THE INVENTION

### 1. Field of the Invention

The present invention relates generally to electromagnetic energy devices and, more particularly, to cutting, treatment and illumination devices that transmit electromagnetic energy toward target surfaces.

### 2. Description of Related Art

Electromagnetic energy devices are employed in a variety of applications. For example, a simple incandescent light may be used to illuminate an area with electromagnetic energy in a form of visible light. Another form of electromagnetic energy, such as a laser beam, may be used to illuminate an area, to identify a target, or to deliver concentrated energy to a target in order to perform various procedures such as melting, cutting, or the like.
Certain medical devices may deliver electromagnetic energy to a target surface such as,
for example, an eye, in order to correct a deficiency in visual acuity. Other medical devices may direct electromagnetic energy toward a surface of a tooth to perform, for example, a cutting operation. Endoscopic devices can be used to enhance visualization of internal parts of, for example, a human body in order to detect and/or remove diseased tissue. Constructions of these devices may vary, while underlying functionalities or goals, including, for example, the provision of efficient operation by supplying optimal illumination without obstructing a user's access or view and/or the provision of reliable operation to ensure reproducibility and favorable procedural results, are often shared.

DE 3911853 discloses devices which may be useful for delivering electromagnetic radiation to a surface, according to the preamble of claim 1.

A need exists in the prior art to efficiently and reliably transmit various types of electromagnetic energy to and from target surfaces in order, for example, to enhance visualization and treatments of the target surfaces.

### SUMMARY OF THE INVENTION

The present invention addresses these needs by providing a laser handpiece that connects to an electromagnetic energy base unit (e.g., a laser base unit). The invention herein disclosed comprises, according to an exemplary embodiment, a laser handpiece having an elongate portion that receives laser energy, illumination light, excitation light, spray water, spray air, and cooling air from a connector that connects to the laser base unit. The handpiece further comprises a handpiece tip formed as an extension of the elongate portion, the handpiece tip being capable of directing laser energy to a target surface. An embodiment of the elongate portion comprises a plurality of optical fibers.

As used herein, "optical fiber" refers to any electromagnetic energy (e.g., light) transmitting medium (e.g., fiber) that is able to transmit light from one end of the fiber to another end of the fiber. The light transmission may be passive or it may include one or more light altering elements to influence the way light is emitted from the optical fiber. Optical fibers can be used to transmit any type of light, including visible light, infrared light, blue light, laser light, and the like. Optical fibers may be hollow or solid, and may include one or more reflectors within bodies of the fibers to control transmission and emission of light from the optical fibers.

Another embodiment of the present invention comprises a laser device that includes a laser base unit, a connector that connects to the laser base unit, and a conduit that connects to the connector. Further, a laser handpiece connects to the conduit, the laser handpiece being capable of receiving laser energy, illumination light, excitation light, spray water, spray air, and cooling air from the laser base unit.

An illumination device in accordance with an aspect of the present invention includes a unitary distal end (output portion) and a split proximal end (input portion). As used herein, "distal end" refers to an end of an illumination device that is closest to a target surface, and "proximal end" refers to an end of an illumination device that is closest to a power source or other source of electromagnetic energy. The illumination device can include a plurality of different sized optical fibers depending on a particular application for which the illumination device is utilized. In illustrative embodiments, and as disclosed herein, the proximal end of the illumination device includes three proximal end members configured to accommodate three sets of optical fibers.

Another illumination device in accordance with an additional aspect of the present invention includes a plurality of sets of optical fibers configured to emit electromagnetic energy from the distal end of the illumination device toward a target surface. The device further may include at least one optical fiber configured to receive electromagnetic energy from the target surface and transmit the energy to the proximal end of the illumination device. The electromagnetic energy transmitted to the proximal end of the illumination device can be used as a signal for further analysis.

In another implementation of the present invention, an illumination device includes a handpiece having a reflector. The reflector is constructed to reflect both laser energy, such as light provided by an erbium laser, and visible light, such as blue light, toward a target surface. In an illustrated embodiment, as disclosed herein, the reflector includes a plurality of mirrors to provide enhanced control of the emission of electromagnetic energy from the optical fibers toward a target surface and of the transmission of electromagnetic energy reflected from the target surface back through the illumination device in the opposite direction.

A further aspect of the present invention can comprise a method of analyzing feedback light from a handpiece in order to monitor integrity of optical components. One implementation of the method comprises receiving feedback light and generating an electrical signal according to the feedback light. The implementation further can provide an error indication when the electrical signal exceeds a predetermined threshold.

For purposes of summarizing the present invention, certain aspects, advantages and novel features of the present invention are described herein. Of course, it is to be understood that not necessarily all such aspects, advantages or features will be embodied in any particular embodiment of the present invention. Additional advantages and aspects of the present invention are apparent in the following detailed description and claims that follow.

### BRIEF DESCRIPTION OF THE FIGURES

FIG. 1 is a pictorial diagram of a delivery system capable of transferring electromagnetic energy to a treatment site in accordance with an example of the present invention;
FIG. 2 is a pictorial diagram illustrating detail of a connector according to an example of the present invention;
FIG. 3 is a perspective diagram of an embodiment of module that may connect to a laser base unit and that may accept the connector illustrated in FIG. 2;
FIG. 4 is a front view of the embodiment of the module illustrated in FIG. 3;
FIG. 5 is a cross-sectional view of the module illustrated in FIG. 4, the cross-section being taken along a line 5-5' of FIG. 4;
FIG. 6 is another cross-sectional view of the module illustrated in FIG. 4, the cross-section being taken along a line 6-6' of FIG. 4;
FIG. 7 is a pictorial diagram of an embodiment of the conduit shown in FIG. 1;
FIG. 8 is a partial cut-away diagram of a handpiece tip in accordance with an example of the present invention;
FIG. 8a is a pictorial diagram of detail of the handpiece tip of FIG. 8 illustrating a mixing chamber for spray air and water;
FIG. 9 is a sectional view of a proximal member of FIG. 7 taken along line 9-9' of FIG. 7;
FIG. 10 is a cross-sectional view of a handpiece tip taken along line 10-10' of FIG. 8;
FIG. 11 is a cross-sectional diagram of another embodiment of the handpiece tip taken along the line 10-10' of FIG. 8;
FIG. 12 is a cross-sectional diagram of another embodiment of the laser handpiece tip taken along line 12-12' of FIG. 8; and
FIG. 13 is a flow diagram describing an implementation of a method of analyzing feedback light in order to monitor integrity of optical components.

### DETAILED DESCRIPTION OF THE PRESENT INVENTION

Reference will now be made in detail to the presently preferred embodiments of the invention, examples of which are illustrated in the accompanying drawings. Wherever possible, the same or similar reference numbers are used in the drawings and the description to refer to the same or like parts. It should be noted that the drawings are in simplified form and are not to precise scale. In reference to the disclosure herein, for purposes of convenience and clarity only, directional terms, such as, top, bottom, left, right, up, down, over, above, below, beneath, rear, and front, are used with respect to the accompanying drawings. Such directional terms should not be construed to limit the scope of the invention in any manner.

Although the disclosure herein refers to certain illustrated embodiments, it is to be understood that these embodiments are presented by way of example and not by way of limitation. The intent of the following detailed description, although discussing exemplary embodiments, is to be construed to cover all modifications of the embodiments as may fall within the scope of the invention as defined by the appended claims. It is to be understood and appreciated that the process steps and structures described herein do not cover a complete process flow for operation of laser devices. The present invention may be practiced in conjunction with various techniques that are conventionally used in the art, and only so much of the commonly practiced process steps are included herein as are necessary to provide an understanding of the present invention. The present invention has applicability in the field of laser devices in general. For illustrative purposes, however, the following description pertains to a medical laser device and a method of operating the medical laser device to perform surgical functions.

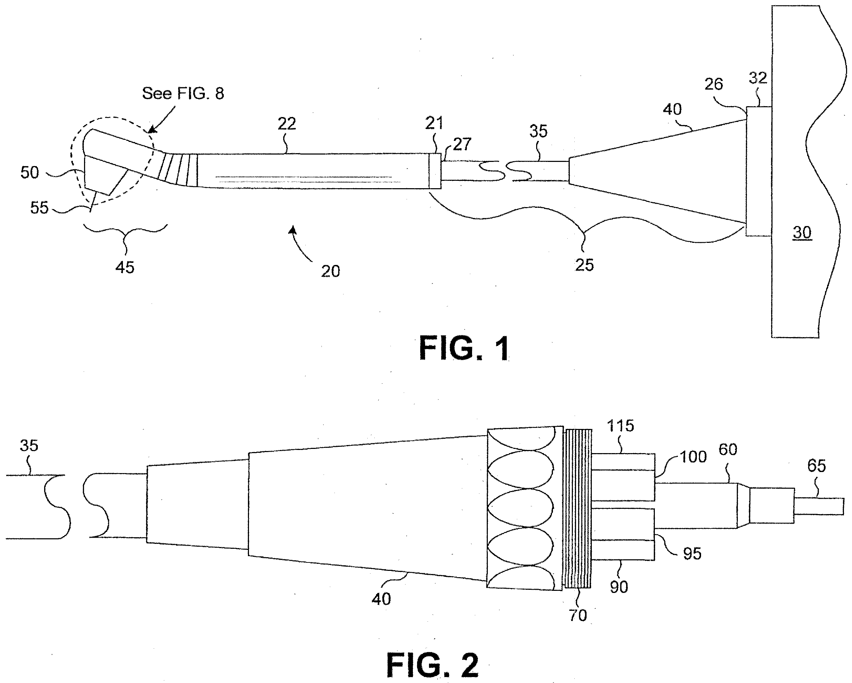

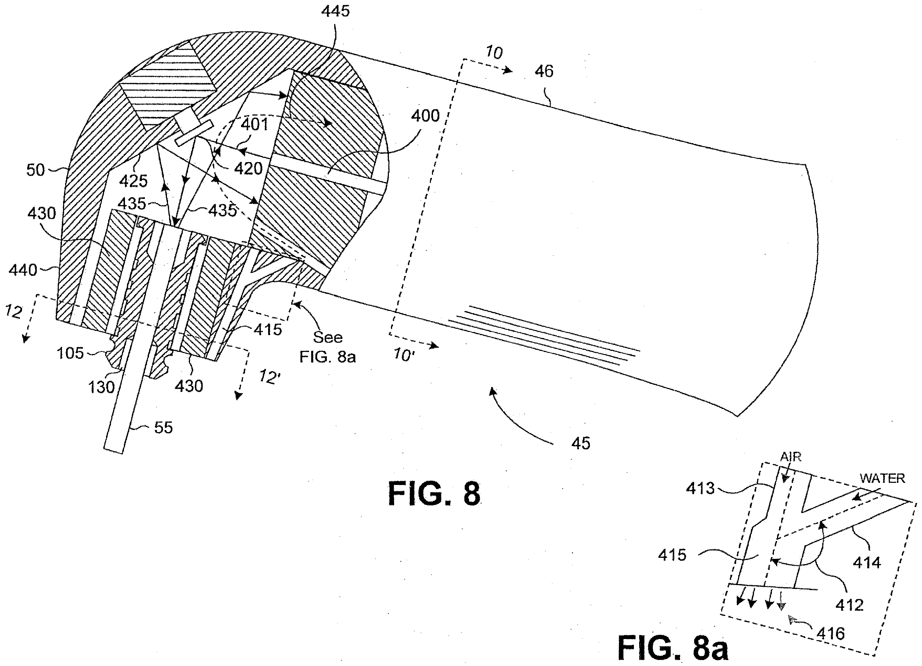

Referring more particularly to the drawings, FIG. 1 is a pictorial diagram of a delivery system capable of transferring laser energy to a treatment site. The illustrated embodiment comprises a laser handpiece 20 that connects to an electromagnetic energy base unit, such as a laser base unit 30, using a linking element 25. The linking element 25 may comprise a conduit 35, which may include one or more optical fibers, tubing for air, tubing for water, and the like. The linking element 25 further may comprise a connector 40 that joins the conduit 35 to the laser base unit 30. The connector 40 may be an identification connector as is described more fully in a U.S. 2006/0142744 entitled IDENTIFICATION CONNECTOR FOR A MEDICAL LASER HANDPIECE. The laser handpiece 20 may comprise an elongate portion 22 and a handpiece tip 45 formed as an extension of the elongate portion 22. The elongate portion 22 may have disposed therein a plurality of optical fibers that may connect to, or that are the same as the optical fibers included in the conduit 35. A proximal (i.e., relatively nearer to the laser base unit 30) portion 21 and a distal (i.e., relatively farther from the laser base unit 30) portion 50 may be disposed at respective proximal and distal ends of the laser handpiece 20. The distal portion 50 has protruding therefrom a fiber tip 55, which is described below in more detail with reference to FIG. 8. As illustrated, the linking element 25 has a first end 26 and a second end 27. The first end 26 couples to a receptacle 32 of the laser base unit 30, and the second end 27 couples to the proximal portion 21 of the laser handpiece 20. The connector 40 may connect mechanically to the laser base unit 30 with a threaded connection to the receptacle 32 that forms part of the laser base unit 30.

An embodiment of a connector 40 is illustrated in greater detail in FIG. 2. The illustrated embodiment comprises a laser beam delivery guide connection 60 that may comprise, for example, a treatment optical fiber 65 capable of transmitting laser energy to the laser handpiece 20 (FIG. 1). The illustrated embodiment further comprises a plurality of ancillary connections comprising, in this example, a feedback connection 115, an illumination light connection 100, a spray air connection 95, and a spray water connection 90, that may connect to the laser base unit 30 (FIG. 1). The plurality of ancillary connections further may comprise connections not visible in FIG. 2 such as an excitation light connection and a cooling air connection.

The embodiment of the connector 40 illustrated in FIG. 2 further comprises a threaded portion 70 that may mate with and thereby provide for connection to the receptacle 32 on the laser base unit 30 (FIG. 1).

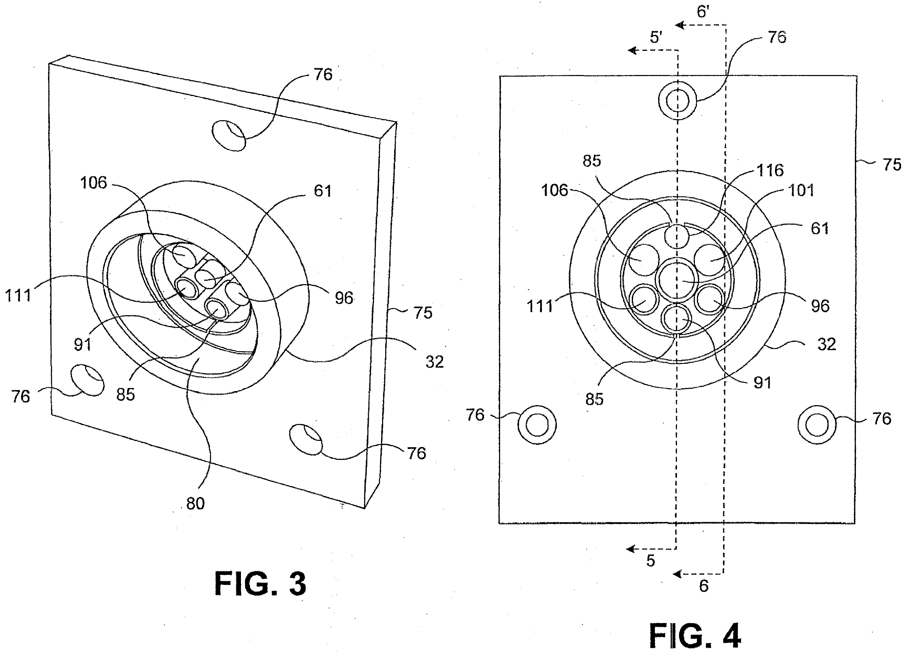

FIG. 3 is a perspective diagram of an embodiment of a module that may connect to, and form a part of, a laser base unit 30 (FIG. 1) and that further may accept connector 40 (FIG. 2). The illustrated embodiment comprises a plate 75 that may fasten to a laser base unit 30 by means of, for example, screws inserted into holes 76. The module comprises a receptacle 32 that may be threaded on an inside surface 80 to mate with threads 70 on the connector 40 (FIG. 2). (Threads are not shown in FIG. 3.) The embodiment of the module further comprises a laser energy coupling 61 mated to the laser beam delivery guide connection 60 (FIG. 2), the laser energy coupling 61 being capable of providing laser energy to the delivery system. The embodiment further comprises a plurality of ancillary couplings including a spray air coupling 96, a spray water coupling 91, a cooling air coupling 111, and an excitation light coupling 106. The embodiment still further comprises a feedback coupling and an illumination light coupling that are not visible in the diagram. One or more key slots 85 may be included to assure that the connector 40 connects to the receptacle 32 in a correct orientation.

FIG. 4 is a front view of the embodiment of the module illustrated in FIG. 3. The view in FIG. 4 illustrates the plate 75 and the holes 76 that may be used to secure the plate module to a laser base unit, such as the laser base unit 30 illustrated in FIG. 1. Further illustrated are the laser energy coupling 61, feedback coupling 116, the illumination light coupling 101, the spray air coupling 96, the spray water coupling 9 1, the cooling air coupling 111, and the excitation light coupling 106. In operation, the spray water coupling 91 mates with and is capable of supplying spray water to the spray water connection 90 in the connector 40 (FIG. 2). Similarly, the spray air coupling 96 mates with and is capable of supplying spray air to the spray air connection 95 in the connector 40. Additionally, the illumination light coupling 101, the excitation light coupling 106, and the cooling air coupling 111 mate with and are capable of supplying, respectively, illumination light to the illumination light connection 100, excitation light to the excitation light connector(not shown), and cooling air to the cooling air connection (not shown) in the connector 40. Further, the feedback coupling 116 mates with and is capable of receiving feedback from the feedback connection 115 in the connector 40. According to an illustrative embodiment, the illumination light coupling 101 and the excitation light coupling 106 couple light from a light-emitting diode (LED) or a leaser light source to, respectively, the illumination light connection 100 and the excitation light connection(not shown). One embodiment employs two white LEDs as a source for illumination light. Also illustrated in FIG. 4 are key slots 85 that may prevent the connector 40 from being connected to the receptacle 32 in an incorrect orientation.

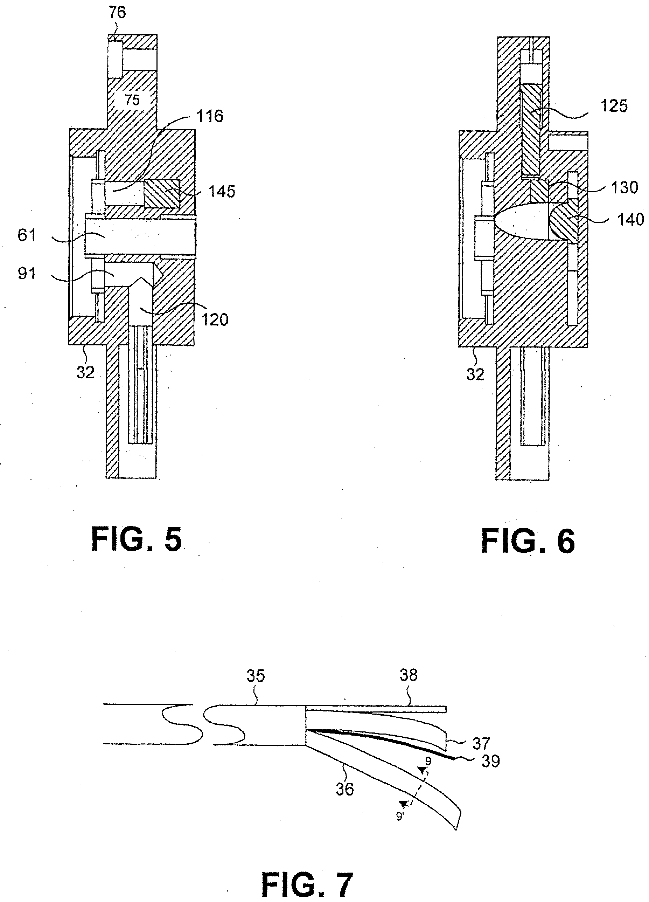

FIG. 5 is a cross-sectional view of the module illustrated in FIGS. 3 and 4. The cross-section is taken along line 5-5' of FIG. 4, the line 5-5' showing cross-sections of the laser energy coupling 61, the feedback coupling 116, and the spray water coupling 91. A water source 120 may supply water to the spray water coupling 91.

FIG. 6 is another cross-sectional view of the module illustrated in FIGS. 3 and 4. The cross-section of FIG 6 is taken along line 6-6' of FIG. 4. The diagram depicts cross-sections of a light source (e.g., an LED 140) that may be capable of supplying light to, for example, one or both of the illumination light coupling 101 (FIG. 4) and the excitation light coupling 106. A pneumatic shutter 125 may control a position of a radiation filter 130 disposed in the laser base unit 30 so that the filter is either inserted or removed from a light path originating with the light source (e.g., the LED 140). For example, one or more pneumatic shutter filters may be provided that enable switching between, for example, blue and white light that is coupled to the illumination light coupling 101 and the excitation light coupling 106 in order to enhance excitation and visualization.

FIG. 7 is a pictorial diagram of an embodiment of the conduit 35 shown in FIG. 1. The illustrated embodiment of the conduit 3 5 comprises a plurality of proximal members, such as, four proximal members comprising first proximal member 36, second proximal member 37, third proximal member 38, and fourth proximal member 39. First, second, and third proximal members 36, 37, and 38 may have hollow interiors configured to accommodate one or more light transmitters or other tubular or elongate structures that have cross-sectional areas less than a cross-sectional area of a hollow interior of the conduit 35. According to one embodiment, first proximal member 36 comprises an illumination fiber, second proximal member 37 comprises an excitation fiber, and third proximal member 38 comprises a feedback fiber. First, second, and third proximal members 36, 37, and 38 may be arranged such that the hollow interior of each proximal member is in communication with a hollow interior of elongate body 22 (FIG. 1). This arrangement provides for a substantially continuous path for the light transmitters to extend from the proximal portion 21 to the distal portion 50 of the laser handpiece 20. The third proximal member 38 may receive feedback (e.g., reflected or scattered light) from the laser handpiece 20 and may transmit the feedback to the laser base unit 30 as is more particularly described below.

The fourth proximal member 39 may comprise a laser energy fiber that receives laser energy derived from an erbium, chromium, yttrium, scandium, gallium, garnet (Er, Cr:YSGG) solid state laser disposed in the laser base unit 30 (FIG. 1). The laser may generate laser energy having a wavelength of approximately 2.78 microns at an average power of about 6 W, a repetition rate of about 20 Hz, and a pulse width of about 150 microseconds. Moreover, the laser energy may further comprise an aiming beam, such as light having a wavelength of about 655 nm and an average power of about 1 mW transmitted in a continuous-wave (CW) mode. The fourth proximal member 39 may be coupled to or may comprise the treatment optical fiber 65 (FIG. 2) that receives laser energy from the laser energy coupling 61 (FIG. 4). The fourth proximal member 39 further may transmit the laser energy received from the laser base unit 30 to the distal portion 50 of the laser handpiece 20 (FIG. 1).

Although the illustrated embodiment is provided with four proximal members, a greater or fewer number of proximal members may be provided in additional embodiments according to, for example, the number of light transmitters provided by the laser base unit 30. In addition, the illustrated embodiment includes first and second proximal members 36 and 37 that have substantially equal diameters and a third proximal member 38 that has a diameter less than either of the diameters of the first and second proximal members 36 and 37. Other configurations of diameters are also contemplated by the present invention. In an exemplary embodiment, the proximal members connect with the connections in the connector 40 illustrated in FIG. 2. For example, the first proximal member 36 may connect with the illumination light connection 100 and the second proximal member 36 may connect with the excitation light connection (not shown). The third proximal member 38 may connect with the feedback connection 115, and the fourth proximal member 39 may connect with the laser beam delivery guide connection 60 and the treatment optical fiber 65. Attachment of the proximal members 36-39 to the connections may be made internal to connector 40 in a manner known or apparent to those skilled in the art in view of this disclosure and is not illustrated in FIGS. 2 and 7.

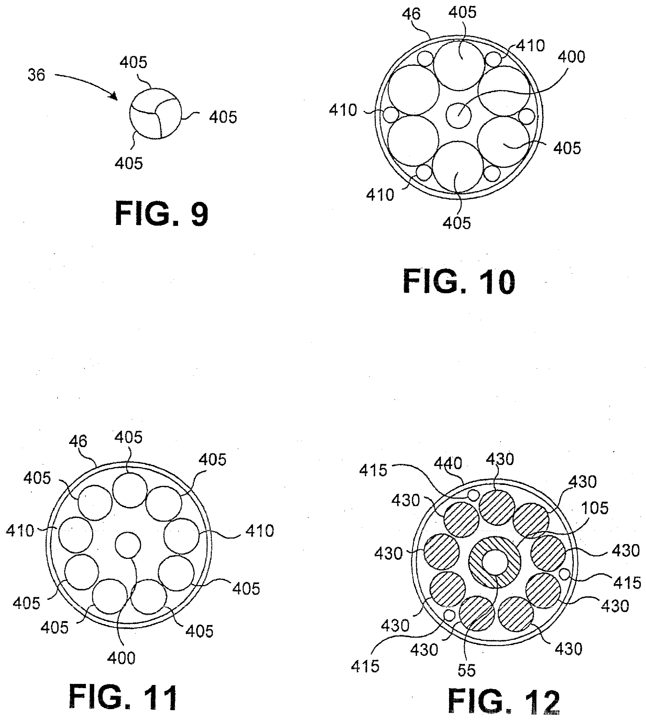

FIG. 8 is a partial cut-away diagram of a handpiece tip 45 (cf. FIG. 1) that couples with the laser base unit 30 by means of the linking element 25 and the elongate portion 22 of the laser handpiece 20. The illustrated embodiment, which is enclosed by an outer surface 46, may receive electromagnetic (e.g., laser) energy, illumination light, excitation light and the like from the laser base unit 30. Typically, the laser energy and light are received by proximal members 36-39 (FIG. 7) as described above and transmitted through waveguides, such as fibers 405 disposed in the elongate portion 22 and the handpiece tip 45 as described below with reference to FIG. 10. For example, illumination light (not shown) may be received by the handpiece tip 45, such as from proximal members 36 and 37 (FIG. 7), carried by fibers 405 (FIG. 10, not shown in FIG. 8), and directed toward a first mirror 425 disposed within the distal portion 50 of the laser handpiece 20. The first mirror 425 in the illustrated embodiment directs illumination light toward a plurality of tip waveguides 430 as is more particularly described below with reference to FIG. 12. Illumination light exiting the tip waveguides 430 may illuminate a target area.

According to one embodiment, concentrated electromagnetic energy, such as laser energy 401, is received (e.g., through fourth proximal member 39 (FIG. 7)) and carried by an internal waveguide such as a treatment optical fiber 400. The laser energy 401 may be directed toward a second mirror 420, which may eclipse at least a part of the first mirror 425 relative to a direction of propagation of the illumination light to the first mirror 425, the second mirror 420 likewise being disposed in the distal portion 50 of the laser handpiece 20. The second mirror 420 may reflect, and thereby direct, the laser energy 401 toward the fiber tip 55. Relative to the concentrated electromagnetic energy (e.g., laser energy 401), the illumination light may comprise an example of additional electromagnetic energy, so described because the illumination light and/or, as described below, excitation light, may comprise electromagnetic energy exhibiting a relatively low power level that is directed to illuminate a portion of a target surface that may, for example, surround a portion of a target surface to which the concentrated electromagnetic energy is directed. The concentrated electromagnetic energy (e.g., laser energy 401) may be directed toward the target surface by the fiber tip 55.

In some embodiments, respective first and second mirrors 425 and 420 may comprise parabolic, toroidal, and/or flat surfaces. FIG. 8 also illustrates a simplified view of a path 445 of cooling air received from a cooling air line (not shown) in the handpiece that may receive cooling air from the cooling air coupling 111 (FIG. 4).

The fiber tip 55 illustrated in FIG. 8 may be encased in a tip ferrule 105 having a distal end. The tip ferrule 105, together with the fiber tip 55, may form a removable, interchangeable unit as is described more fully in U.S. 2007/0128576 entitled, OUTPUT ATTACHMENTS CODED FOR USE WITH ELECTROMAGNETIC-ENERGY PROCEDURAL DEVICE.

FIG. 9 is a cross-sectional view of first proximal member 36 taken along line 9-9' of FIG. 7 demonstrating that first proximal member 36 (as well as, optionally, second proximal member 37) may comprise three optical fibers 405 substantially fused together to define a unitary light emitting assembly or waveguide. In modified embodiments, the three optical fibers 405 may be joined by other means or not joined. According to other embodiments, one or more of the proximal members, such as the second proximal member 37, can include different numbers of optical fibers 405. In an illustrated embodiment, the second proximal member 37 can include six optical fibers 405 (FIG. 9) that begin to separate and eventually (e.g., at line 10-10' in FIG. 8) surround a laser energy waveguide, such as treatment optical fiber 400, as illustrated in a cross-sectional view of FIG. 10 taken along line 10-10' of FIG. 8 in the handpiece tip 45. In another exemplary embodiment, the second proximal member 37 can include three optical fibers 405 (FIG. 9) and the first proximal member 36 can include three optical fibers 405 (FIG. 9), all six of which begin to separate and eventually (e.g., at line 10-10' in FIG. 8) surround a laser energy waveguide, such as treatment optical fiber 400 in the handpiece tip 45.

The third proximal member 38 may include six relatively smaller fibers 410, as likewise is shown in the cross-sectional view of FIG. 10. Additional waveguides, such as additional fibers 410, may be disposed within the outer surface 46 and, further, may be configured to receive feedback from a target surface. For example, feedback may comprise scattered light 435 (FIG. 8) received from the fiber tip 55 in a manner more particularly described below. The scattered light 435 (i.e., feedback light) may be transmitted by third proximal member 38 (FIG. 7) to the laser base unit 30 (FIG. 1). Fibers 410 are illustrated in FIG. 10 as being separate from each other, but in additional embodiments two or more of the fibers 410 can be fused or otherwise joined together. Fibers 405 and 410 can be manufactured from plastic using conventional techniques, such as extrusion and the like.

FIG. 11 is a cross-sectional diagram of another embodiment of the handpiece tip 45, the cross-section being taken along line 10-10' in FIG. 8. FIG. 11 depicts a laser energy waveguide, such as treatment optical fiber 400 surrounded by illumination waveguides, such as fibers 405, and feedback waveguides, such as fibers 410, all of which are disposed within outer surface 46. In a manner similar to that described above with reference to FIG. 10, the illumination waveguides, such as fibers 405 may receive light energy from the laser base unit 30 (FIG. 1) by way of illumination light coupling 101 (FIG. 4), illumination light connection 100 (FIG. 2), and, for example, proximal members 36 and/or 37 (FIG. 7); and fibers 405 may direct the light to the distal portion 50 of the laser handpiece 20 (FIG. 8).

In certain implementations involving, for example, caries detection, as disclosed in a U.S. Application filed August 12, 2005 and entitled CARIES DETECTION USING TIMING DIFFERENTAILS BETWEEN EXCITATION AND RETURN PULSES, the entire contents of which are incorporated herein by reference, fibers 405 further may function as both illumination and excitation waveguides. Feedback waveguides, such as fibers 410, may receive feedback light from the fiber tip 55 (FIG. 8) and may transmit the feedback light to third proximal member 38, which couples to or comprises feedback connection 115. The feedback light may be received by the feedback coupling 116, which transmits the light to a feedback detector 145 (FIG. 5) disposed in the laser base unit 30 (FIG. 1). In other embodiments, such as described more fully in the above-referenced U.S. 2006/0142744 entitled IDENTIFICATION CONNECTOR FOR A MEDICAL LASER HANDPIECE, the laser base unit 30 may additionally supply spray air, spray water, and cooling air to the laser handpiece 20.

FIG. 12 is a cross-sectional diagram of another embodiment of the laser handpiece tip 45 taken along line 12-12' of FIG. 8. This embodiment illustrates a fiber tip 55 surrounded by a tip ferrule or sleeve 105, and, optionally, glue that fills a cavity 130 around the fiber tip 55 to hold the fiber tip 55 in place. Tip waveguides 430 may receive illumination light from second mirror 425 (FIG. 8) and direct the illumination light to a target. In some embodiments, fluid outputs 415, which are disposed in the handpiece tip 45, may carry, for example, air and water. More particularly, illumination light exiting from the illumination fibers 405 (cf. FIG. 11) is reflected by second mirror 425 (FIG. 8) into the tip waveguides 430 (FIGS. 8 and 12). While a portion of this illumination light may also be reflected by second mirror 425 (FIG. 8) into fiber tip 55, fiber tip 55 receives, primarily, a relatively high level of laser energy 401 from treatment optical fiber 400 (cf. FIG. 11), which laser energy, as presently embodied, comprises radiation including both a cutting beam and an aiming beam. In a representative embodiment, illumination light from the illumination fibers 405 that exits the tip waveguides 430 is white light of variable intensity (e.g., adjustable by a user) for facilitating viewing and close examination of individual places of a target surface, such as a tooth. For example, a cavity in a tooth may be closely examined and treated with the aid of light from a plurality of tip waveguides 430.

A detailed illustration of an embodiment of a chamber for mixing spray air and spray water in the handpiece tip 45 is shown in FIG. 8a. As illustrated, the mixing chamber comprises an air intake 413 connected to, for example, tubing (e.g., a spray air line, not shown) that connects to and receives air from, the spray air connection 95 in the connector 40 (FIG. 2). Similarly, a water intake 414 may connect to tubing (also not shown) that connects to and receives water from the spray water connection 90 in the connector 40 (FIG. 2). The air intake 413 and the water intake 414, which may have circular cross-sections about 250 µm in diameter, join at an angle 412 that may approximate 110° in a typical embodiment. Mixing may occur in a neighborhood where the air intake 413 and water intake 414 join, and a spray (e.g., atomized) mixture 416 of water and air may be ejected through a fluid output 415. The embodiment illustrated in FIG. 12 depicts three fluid outputs 415. These fluid outputs may , for example, correspond to, comprise parts of, or comprise substantially all of, any of fluid outputs described in U.S. 2005/0256517 entitled ELECTROMAGNETICALLY INDUCED TREATMENT DEVICES AND METHODS, the entire contents of which are incorporated herein by reference to the extent compatible, or, in other embodiments, structures described in the referenced provisional patent application may be modified to be compatible with the present invention. The fluid outputs 415 may, as illustrated in FIGS. 8 and 12, have circular cross-sections measuring about 350 µm in diameter.

Scattering of light as described above with reference to FIG. 7 can be detected and analyzed to monitor various conditions. For example, scattering of an aiming beam can be detected and analyzed to monitor, for example, integrity of optical components that transmit the cutting and aiming beams. In typical implementations the aiming beam may cause little to no reflection back into the feedback fibers 410. However, if any components (such as, for example, second mirror 420 or fiber tip 55) is damaged, scattering of the aiming beam light (which may be red in exemplary embodiments) may occur. Scattered light 435 (FIG. 8) may be directed by the second mirror 425 into feedback fibers 410 that may convey the scattered light to the laser base unit 30 (FIG. 1).

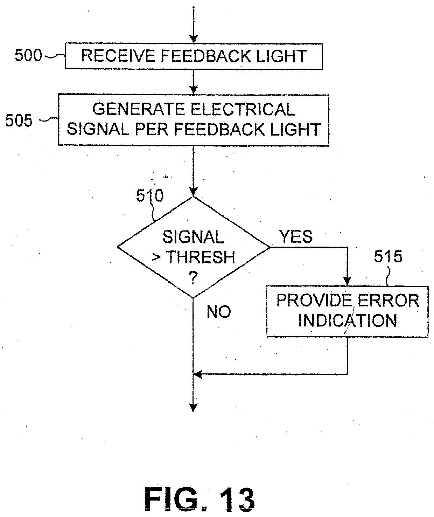

FIG. 13 is a flow diagram describing an implementation of a method of analyzing light, such as feedback light, in order to monitor integrity of optical components. This implementation of the method receives feedback light (i.e., scattered light) at step 500. For example, the feedback light may be received by a light discerning device, such as photo detector 145 (FIG. 5), that forms an electrical signal from the feedback light at step 505. Detection of scattered aiming beam light having an intensity above a predetermined threshold can trigger the laser base unit 30 or other machinery to provide an indication of error or potential error. According to the implementation of the method illustrated in FIG. 13, a magnitude of the electrical signal is compared with the predetermined threshold at step 510. An error indication is provided at step 515 if the electrical signal exceeds the predetermined threshold. That is, a magnitude of detected scattered light 435 from the feedback fibers 410 and/or relative magnitudes of detected scattered light among the various feedback fibers 410 can be automatically analyzed and compared with predetermined optical-component failure criteria to provide additional information to a user regarding a type, location and/or severity of the potential optical-component problem. A feedback display can be provided on a monitor of the laser base unit 30 (e.g., a color of blue) to indicate one or more of the above-described indications or parameters.

The present invention contemplates constructions and uses of visual feedback implements (e.g., cameras) as described in, for example, U.S 2006/0281042 entitled ELECTROMAGNETIC RADIATION EMITTING TOOTHBRUSH AND DENTIFRICE SYSTEM, and U.S. Provisional Application No. 60/687,991, filed June 6, 2005 and entitled METHODS FOR TREATING EYE CONDITIONS, on (e.g., attached) or in a vicinity of (e.g., on or near, attached or not, output ends) of electromagnetic energy output devices (e.g., lasers and dental lasers), wherein such output devices, constructions and uses can be, in whole or in part, including any associated methods, modifications, combinations, permutations, and alterations of any constructions(s) or use(s) described or referenced herein or recognizable as included or includable in view of that described or referenced herein by one skilled in the art, to the extent not mutually exclusive, as described in U.S. 2006/0241574 entitled ELECTROMAGNETIC ENERGY DISTRIBUTIONS FOR ELECTROMAGNETICALLY INDUCED DISRUPTIVE CUTTING, U.S. 2005/0283143 entitled TISSUE REMOVER AND METHOD, U.S. 2006/0142745 entitled DUAL PULSE-WIDTH MEDICAL LASER WITH PRESETS, U.S. 2007/0016176 entitled LASER HANDPIECE ARCHITECTURE AND METHODS, and U.S. Application No. 09/848,010, filed May 2, 2001 and entitled DERMATOLOGICAL CUTTING AND ABLATING DEVICE. In some embodiments, the sensor may comprise one or more visual feedback implements. The visual feedback implement can be used, for example, (a) in a form that is integrated into a handpiece or output end of an electromagnetic energy output device, (b) in a form that is attached to the handpiece or electromagnetic energy output device, or (c) in conjunction with (e.g., not attached to) the handpiece or electromagnetic energy output device, wherein such handpieces and devices can facilitate cutting, ablating, treatments, and the like. Treatments can include low-level light treatments such as described in the above-referenced U.S. Provisional Application No. 60/687,991 entitled METHODS FOR TREATING EYE CONDITIONS and U.S. 2007/0208404 entitled TISSUE TREATMENT DEVICE AND METHOD.

For example, one implementations may be useful for, among other things, optimizing, monitoring, or maximizing a cutting effect of an electromagnetic energy emitting device, such as a laser handpiece. The laser output can be directed, for example, into fluid (e.g., an air and/or water spray or an atomized distribution of fluid particles from a water connection and/or a spray connection near an output end of the handpiece) that is emitted from the handpiece above a target surface. An apparatus including corresponding structure for directing electromagnetic energy into an atomized distribution of fluid particles above a target surface is disclosed, for example, in the above-referenced U.S. Patent No. 5,574,247. Large amounts of laser energy, for example, can be imparted into the fluid (e.g., atomized fluid particles), which can comprise water, to thereby expand the fluid (e.g., fluid particles) and apply disruptive (e.g., mechanical) cutting forces to the target surface. During a procedure, such as an oral procedure where access and visibility are limited, careful and close-up monitoring by way of a visual feedback implement of (a) interactions between the electromagnetic energy and the fluid (e.g., above the target surface) and/or (b) cutting, ablating, treating or other impartations of disruptive surfaces to the target surface, can improve a quality of the procedure.

In certain embodiments, visualization optical fibers (e.g., a coherent fiber bundle) can be provided that are configured to transmit light from the distal portion 50 to the proximal portion 21, for routing images (e.g., working-surface images) acquired at or in a vicinity of the distal portion by a visual feedback implement. According to some embodiments, the visual feedback implement can comprise an image-acquisition device (e.g., CCD or CMOS camera) for obtaining or processing images from the distal portion. The visual feedback implement can be built-in or attached (e.g., removably attached) to the handpiece and, further, can be disposed at various locations on or in connection with the handpiece between the proximal portion and distal portion, or proximally of the proximal portion. According to this and any of the other embodiments described herein, one or more of the optical fibers described herein and the visualization optical fibers can be arranged, for example, outside of the handpiece envelope. A few applications for the presently-described visual feedback implement may include periodontal pockets (e.g., diagnostic and treatment), endodontics (e.g., visualization of canals), micro-dentistry, tunnel preparations, caries detection and treatment, bacteria visualization and treatment, general dentistry, and airborne-agent and gas detection applications as described in the above-referenced U.S. Application No. 11/438,091.

According to another embodiment of the present invention, electromagnetic radiation (e.g., one or more of blue light, white light, infrared light, a laser beam, reflected/scattered light, fluorescent light, and the like, in any combination) may be transmitted in one or both directions through one or more of the fibers described herein (e.g., feedback, illumination, excitation, treatment), in any combination. Outgoing and incoming beams of electromagnetic radiation can be separated or split, for example, according to one or more characteristics thereof, at the proximal portion or laser base unit using a beam splitter, such as a wavelength-selective beam splitter (not shown), in a manner known to those skilled in the art.

In a representative embodiment, the fluid outputs 415 (FIG. 12) are spaced at zero (a first reference), one hundred twenty, and two hundred forty degrees. In another embodiment, the six illumination/excitation fibers 405 and three feedback fibers 410 (FIG. 11) are optically aligned with and coupled via second mirror 425 on, for example, a one-to-one basis, to nine tip waveguides 430 (FIGS. 8 and12). For example, if nine elements (e.g., six illumination/excitation fibers 405 and three feedback fibers 410) are evenly spaced and disposed at zero (a second reference, which may be the same as or different from the first reference), forty, eighty, one hundred twenty, one hundred sixty, two hundred, two hundred forty, two hundred eighty, and three hundred twenty degrees, then nine tip waveguides 430 may likewise be evenly spaced and disposed at zero, forty, eighty, one hundred twenty, one hundred sixty, two hundred, two hundred forty, two hundred eighty, and three hundred twenty degrees. In another embodiment wherein, for example, the tip waveguides 430 are arranged in relatively closely-spaced groups of three with each group being disposed between two fluid outputs, the tip waveguides 430 may be disposed at, for example, about zero, thirty-five, seventy, one hundred twenty, one hundred fifty-five, one hundred ninety, two hundred forty, two hundred seventy-five, and three hundred ten degrees. In one such embodiment, the tip waveguides 430 may likewise be disposed at about zero, thirty-five, seventy, one hundred twenty, one hundred fifty-five, one hundred ninety, two hundred forty, two hundred seventy-five, and three hundred ten degrees. Further, in such an embodiment, the fluid outputs may be disposed between the groups of tip waveguides at about ninety-five, two hundred fifteen, and three hundred thirty-five degrees.

The cross-sectional views of FIGS. 10 and 11 may alternatively (or additionally), without being changed, correspond to cross-sectional lines 10-10' taken in FIG. 8 closer to (or next to) first and second mirrors 425 and 420 to elucidate corresponding structure that outputs radiation distally onto the first mirror 425 and the second mirror 420. The diameters of illumination/excitation fibers 405 and feedback fibers 410 may be different as illustrated in FIG. 10 or the diameters may be the same or substantially the same as shown in FIG. 11. In an exemplary embodiment, the illumination/excitation fibers 405 and feedback fibers 410 in FIG. 11 comprise plastic constructions with diameters of about 1 mm, and the tip waveguides 430 in FIGS. 8 and 12 comprise sapphire constructions with diameters of about 0.9 mm.

By way of the disclosure herein, a handpiece has been described that utilizes electromagnetic energy to affect a target surface. In the case of dental procedures using laser energy, the handpiece can include an optical fiber for transmitting laser energy to a target surface for treating (e.g., ablating) a dental structure, such as a tooth, a plurality of optical fibers for transmitting light (e.g., blue light) for illumination, curing, whitening, and/or diagnostics of a tooth, a plurality of optical fibers for transmitting light (e.g., white light) to a tooth to provide illumination of the target surface, and a plurality of optical fibers for transmitting light from the target surface back to a sensor for analysis. In the illustrated embodiment, the optical fibers that transmit blue light also transmit white light. In accordance with one aspect of the invention herein disclosed, a handpiece comprises an illumination tube having a feedback signal end and a double mirror handpiece.

In certain embodiments, the methods and apparatuses of the above embodiments can be configured and implemented for use, to the extent compatible and/or not mutually exclusive, with existing technologies including any of the above-referenced apparatuses and methods. Corresponding or related structure and methods are described in the following patents assigned to BioLase Technology, Inc., which a corresponding or related structure (and modifications thereof) in the following patents may be (i) operable with, (ii) modified by one skilled in the art to be operable with, and/or (iii) implemented/used with or in combination with any part(s) of, the present invention according to this disclosure, that/those of the patents, and the knowledge and judgment of one skilled in the art: U.S. Patent No. 5,741,247; U.S. Patent No. 5,785,521; U.S. Patent No. 5,968,037; U.S. Patent No. 6,086,367; U.S. Patent No. 6,231,567; U.S. Patent No. 6,254,597, U.S. Patent No. 6, 288,499; U.S. Patent No. 6,350,123; U.S. Patent No. 6,389,193; U.S. Patent No. 6,544,256; U.S. Patent No. 6,561,803; U.S. Patent No. 6,567,582; U.S. Patent No. 6,610,053; U.S. Patent No. 6,616,447; U.S. Patent No. 6,616,451; U.S. Patent No. 6,669,685; and U.S. Patent No. 6,744,790, all of which are commonly assigned.

One implementation may be useful for tailoring, optimizing or maximizing an effect (e.g., cutting or ablating) of a laser. The laser output (e.g., from a power fiber) can be directed, for example, into fluid (e.g., an air and/or water spray or an atomized distribution of fluid particles from a water connection and/or a spray connection near an output end of the handpiece) that is emitted from a fluid output of the handpiece above a target surface (e.g., one or more of tooth, bone, cartilage and soft tissue). The fluid output may comprise a plurality of fluid outputs, concentrically arranged around a power fiber, as described in, for example, U.S. Application No. 11/042,824 and U.S. Provisional Application No. 60/601,415. The power fiber may comprise, for example, a treatment optical fiber, and in various implementations may be coupled to an electromagnetic energy source comprising one or more of a wavelength within a range from about 2.69 to about 2.80 microns and a wavelength of about 2.94 microns. In certain implementations the power fiber may be coupled to one or more of an Er:YAG laser, an Er:YSGG laser, an Er, Cr:YSGG laser and a CTE:YAG laser, and in particular instances may be coupled to one of an Er, Cr:YSGG solid state laser having a wavelength of about 2.789 microns and an Er:YAG solid state laser having a wavelength of about 2.940 microns. An apparatus including corresponding structure for directing electromagnetic energy into an atomized distribution of fluid particles above a target surface is disclosed in the above-referenced U.S. Patent No. 5,574,247, which describes the impartation of laser energy into fluid particles to thereby apply disruptive forces to the target surface.
While this invention has been described with respect to various specific examples and embodiments, it is to be understood that the invention is not limited thereto and that it can be variously practiced. Multiple variations and modification to the disclosed embodiments will occur, to the extent not mutually exclusive, to those skilled in the art upon consideration of the foregoing description. Additionally, other combinations, omissions, substitutions and modifications will be apparent to the skilled artisan in view of the disclosure herein. Accordingly, the present invention should not be limited by the disclosed embodiments, but is to be defined by reference to the appended claims.

The following examples are also disclosed:

### Examples

1. An electromagnetic energy handpiece that connects to an electromagnetic energy base unit, the handpiece comprising:
   an elongate portion coupled to receive concentrated electromagnetic energy and additional electromagnetic energy from a connector that is connectable to the electromagnetic energy base unit;
   a handpiece tip formed as an extension of the elongate portion, the handpiece tip being capable of directing electromagnetic energy to a target surface;
   a first mirror disposed in a general vicinity between opposing ends of the handpiece and the elongate portion and being capable of directing the additional electromagnetic energy through at least part of the handpiece tip and to the target surface; and
   a second mirror eclipsing at least a part of the first mirror, relative to a direction of propagation of additional electromagnetic energy to the first mirror, and being capable of directing the concentrated electromagnetic energy through at least part of the handpiece tip and to the target surface.
2. The electromagnetic energy handpiece as set forth in example 1, wherein:
   the first mirror is capable of receiving additional electromagnetic energy from one or more conduits and directing the additional electromagnetic energy to one or more tip waveguides of the handpiece tip, the tip waveguides directing the additional electromagnetic energy toward the target surface; and
   the second mirror is capable of receiving concentrated electromagnetic energy from an electromagnetic energy conduit and directing concentrated the electromagnetic energy to a fiber tip of the handpiece tip, the fiber tip directing the concentrated electromagnetic energy toward the target surface.
3. The electromagnetic energy handpiece as set forth in example 1, wherein:
   the elongate portion is coupled to receive spray water from the connector; and
   the elongate portion comprises a spray water line coupled to receive the spray water and to route the spray water through at least part of the elongate portion to the handpiece tip.
4. The electromagnetic energy handpiece as set forth in example 1, wherein the additional electromagnetic energy comprises illumination light and excitation light that are both routed through at least part of the elongate portion to the handpiece tip.
5. The electromagnetic energy handpiece as set forth in example 4, wherein the illumination light and the excitation light are routed through at least part of the elongate portion by way of an illumination fiber and an excitation fiber.
6. The electromagnetic energy handpiece as set forth in example 4, wherein the elongate handpiece further receives spray water from the connector.
7. The electromagnetic energy handpiece as set forth in example 6, wherein the elongate handpiece further receives air from the connector.
8. The electromagnetic energy handpiece as set forth in example 7, wherein the air is supplied to the elongate handpiece by way of a spray air line and a cooling air line.
9. The electromagnetic energy handpiece as set forth in example 1, wherein the elongate portion comprises:
   an electromagnetic energy fiber capable of receiving concentrated electromagnetic energy from the connector;
   an illumination fiber capable of receiving a portion of the additional electromagnetic energy as illumination light;
   an excitation fiber capable of receiving another portion of the additional electromagnetic energy as excitation light; and
   a feedback fiber capable of receiving feedback light from the handpiece tip.
10. The electromagnetic energy handpiece as set forth in example 9, the first mirror being disposed within the handpiece tip and being capable of directing the additional light as illumination light into a plurality of tip waveguides, the second mirror being disposed within the handpiece tip and being capable of directing the concentrated electromagnetic energy into a fiber tip, and the tip waveguides being capable of receiving illumination and excitation light from the first mirror and directing the illumination light to the target surface and receiving reflected light from the target surface and directing the reflected light to the first mirror.
11. The electromagnetic energy handpiece as set forth in example 10, further comprising at least one feedback fiber disposed within the handpiece tip and within the elongate portion, wherein the feedback fiber is capable of receiving reflected light from the first mirror and directing the feedback light to the connector.
12. The electromagnetic energy handpiece as set forth in example 11, wherein the electromagnetic energy base unit comprises a photo detector capable of receiving the feedback light from the connector and providing a feedback display according to one of an error condition and a potential error condition in optical components of the electromagnetic energy handpiece.
13. The electromagnetic energy handpiece as set forth in example 1, further comprising:
   first tubing disposed within the elongate portion and the handpiece tip, the first tubing being capable of receiving air from the connector; and
   second tubing disposed within the elongate portion and the handpiece tip, the second tubing being capable of receiving water from the connector.
14. The electromagnetic energy handpiece as set forth in example 13, further comprising one or more mixing chambers, each mixing chamber having two inputs and a fluid output, the two inputs comprising:
   a first input capable of receiving air from first tubing; and
   a second input capable of receiving water from second tubing, wherein the air and water are mixed within the mixing chamber, and a mixture of air and water is expelled from the fluid output.
15. The electromagnetic energy handpiece as set forth in example 13, the one or more mixing chambers comprising three mixing chambers.
16. An electromagnetic energy device comprising:
   an electromagnetic energy base unit;
   a connector that connects to the electromagnetic energy base unit;
   a conduit that connects to the connector; and
   an electromagnetic energy handpiece that connects to the conduit, wherein the electromagnetic energy handpiece is capable of receiving electromagnetic energy, one or more of illumination light and excitation light having a propagation path that envelops at least a part of a propagation path of the electromagnetic energy within the electromagnetic energy handpiece, and fluid from the electromagnetic energy base unit.
17. The electromagnetic energy device as set forth in example 16, wherein the fluid comprises spray water, spray air, and cooling air.
18. The electromagnetic energy device as set forth in example 16, wherein the electromagnetic energy handpiece comprises a handpiece tip, the handpiece tip comprising:
   a plurality of mirrors;
   a fiber tip; and
   a plurality of tip waveguides, capable of receiving one or more of electromagnetic energy, illumination light, and excitation light, and directing the one or more of electromagnetic energy, illumination light, and excitation light to a target surface.
19. The electromagnetic energy device as set forth in example 18, wherein the handpiece tip comprises a housing having disposed therein the plurality of tip waveguides, the fiber tip, and a plurality of fluid outputs.
20. The electromagnetic energy device as set forth in example 19, wherein the plurality of tip waveguides and the plurality of fluid outputs are circularly disposed around the fiber tip.
21. The electromagnetic energy device as set forth in example 20, wherein:
   the plurality of tip waveguides comprises nine tip waveguides separated by about forty degrees; and
   the plurality of fluid outputs comprises three fluid outputs separated by about one hundred twenty degrees.
22. The electromagnetic energy device as set forth in example 21, wherein:
   the nine tip waveguides are disposed with respect to a reference at zero, forty, eighty, one hundred twenty, one hundred sixty, two hundred, two hundred forty, two hundred eighty, and three hundred twenty degrees; and
   the three fluid outputs are disposed with respect to the reference at one hundred, two hundred twenty, and three hundred forty degrees.
23. The electromagnetic energy device as set forth in example 18, wherein the handpiece tip comprises a housing having disposed therein transparent material capable of transmitting light.
24. The electromagnetic energy device as set forth in example 23, wherein the transparent material comprises one of transparent plastic, sapphire, and quartz.
25. A method of analyzing feedback light from a medical electromagnetic energy handpiece, thereby monitoring integrity of optical components, the method comprising:
   receiving feedback light into the medical electromagnetic energy handpiece;
   generating an electrical signal according to the feedback light; and
   providing an error indication when the electrical signal exceeds a predetermined threshold.
26. The method as set forth in example 25, wherein the providing of an error indication comprises generating a display on a monitor of an electromagnetic energy base unit.
27. A laser handpiece having a proximal end and a distal end, the laser handpiece comprising:
   a power fiber extending from the proximal end to the distal end;
   a plurality of first optical fibers concentrically arranged around the power fiber and extending from the proximal end to the distal end, the plurality of first optical fibers being capable of receiving a first type of electromagnetic energy from the proximal end and of outputting the first type of electromagnetic energy at the distal end; and
   a plurality of second optical fibers concentrically arranged around the power fiber and extending from the proximal end to the distal end, the plurality of second optical fibers being capable of receiving a second type of electromagnetic energy from the distal end and of directing the second type of electromagnetic energy to the proximal end.
28. The laser handpiece as set forth in example 27, further comprising:
   a plurality of third optical fibers extending from the proximal end to the distal end, the plurality of third optical fibers being capable of receiving a third type of electromagnetic energy from the distal end and of directing the third type of electromagnetic energy to the proximal end; and
   a camera coupled to receive the third type of electromagnetic energy from at least part of the plurality of third optical fibers.
29. The laser handpiece as set forth in example 28, wherein the second type of electromagnetic energy is substantially the same as the third type of electromagnetic energy.
30. The laser handpiece as set forth in example 27, further comprising an electromagnetic energy sensor coupled to receive electromagnetic energy from at least part of the plurality of second optical fibers.
31. The laser handpiece as set forth in example 30, wherein the electromagnetic energy sensor includes a camera coupled to receive electromagnetic energy from at least part of the plurality of second optical fibers.
32. The laser handpiece as set forth in example 30, wherein:
   the electromagnetic energy sensor is coupled to receive the second type of electromagnetic energy from at least part of the plurality of second optical fibers; and
   the laser handpiece further comprises a camera that is coupled to receive the second type of electromagnetic energy from at least part of the plurality of second optical fibers.
33. The laser handpiece as set forth in example 27, wherein the plurality of first optical fibers is capable of receiving electromagnetic energy comprising one or more of visible light, infrared light, blue light, and laser light.
34. The laser handpiece as set forth in example 27, further comprising an electromagnetic energy sensor coupled to receive electromagnetic energy from the plurality of second optical fibers.
35. The laser handpiece as set forth in example 34, wherein the electromagnetic energy sensor is coupled to receive the second type of electromagnetic energy from at least part of the plurality of second optical fibers.
36. The laser handpiece as set forth in example 34, wherein the laser handpiece includes at least one light altering element capable of' influencing a transmission of electromagnetic energy by the plurality of first optical fibers.
37. The laser handpiece as set forth in example 36, wherein the at least one light altering element comprises at least one optical filter.
38. The laser handpiece as set forth in example 37, wherein the at least one optical filter is structured to convert blue light into white light.
39. The laser handpiece as set forth in example 27, further comprising a beam splitter coupled to one or more of (a) at least part of the plurality of first optical fibers and (b) at least part of the plurality of second optical fibers.
40. The laser handpiece as set forth in example 39, further comprising an electromagnetic energy sensor coupled to receive electromagnetic energy from at least part of the plurality of second optical fibers.
41. The laser handpiece as set forth in example 39, further comprising a camera coupled to receive electromagnetic energy from at least part of the plurality of second optical fibers.
42. The laser handpiece as set forth in example 41, further comprising an electromagnetic energy sensor coupled to receive electromagnetic energy from at least part of the plurality of second optical fibers.
43. The laser handpiece as set forth in example 42, wherein:
   the electromagnetic energy sensor is coupled to receive the second type of electromagnetic energy from at least part of the plurality of second optical fibers; and
   the camera is coupled to receive the second type of electromagnetic energy from at least part of the plurality of second optical fibers.
44. The laser handpiece as set forth in example 39, further comprising:
   a plurality of third optical fibers extending from the proximal end to the distal end, the plurality of third optical fibers being capable of receiving a third type of electromagnetic energy from the distal end and of directing the third type of electromagnetic energy to the proximal end; and
   a camera coupled to receive the third type of electromagnetic energy from at least part of the plurality of third optical fibers.
45. An apparatus, comprising:
   a laser handpiece having a proximal end and a distal end, the laser handpiece being capable of transmitting concentrated infrared electromagnetic energy and relatively less-concentrated visible electromagnetic energy from the proximal end to the distal end, whereby the less-concentrated visible electromagnetic energy is concentrically disposed around the concentrated infrared electromagnetic energy; and
   a handpiece tip disposed at the distal end of the laser handpiece, the handpiece tip being capable of receiving the concentrated infrared and less-concentrated visible electromagnetic energies from the distal end of the laser handpiece and of directing the electromagnetic energy to a target.
46. The apparatus as set forth in example 45, wherein the handpiece tip comprises:
   a treatment end connected to and disposed at an angle relative to a longitudinal axis of the laser handpiece;
   a tip ferrule insertable into the treatment end, the tip ferrule comprising a distal end; and
   a fiber tip insertable into the tip ferrule, the fiber tip being capable of receiving electromagnetic energy from the distal end of the laser handpiece.
47. The apparatus as set forth in example 45, wherein the laser handpiece comprises:
   a first plurality of optical fibers capable of receiving the concentrated infrared and less-concentrated visible electromagnetic energies at the proximal end and of directing the received electromagnetic energies to the handpiece tip; and
   a second plurality of optical fibers capable of receiving less-concentrated visible electromagnetic energy, which is reflected from a target back into the apparatus, from the handpiece tip and of directing the received less-concentrated visible electromagnetic energy to the proximal end of the laser handpiece.
48. The apparatus as set forth in example 45, wherein during operation the handpiece tip is capable of rotating about an axis of the laser handpiece.
49. The apparatus as set forth in example 48. wherein the handpiece tip comprises a plurality of reflectors capable of directing electromagnetic energy from the distal end of the laser handpiece to a target independent of an angle of rotation of the handpiece tip.
50. A laser handpiece, comprising:
   an elongate body having a distal end and a proximal end;
   a power light transmitter;
   a first plurality of light transmitters disposed within the elongate body around the power transmitter, the first plurality of light transmitters being configured to transmit electromagnetic energy from the proximal end to the distal end;
   a second plurality of light transmitters disposed within the elongate body around the power transmitter, the second plurality of light transmitters being configured to transmit electromagnetic energy from the distal end to the proximal end; and
   a light sensor coupled to receive light from the second plurality of light transmitters at the proximal end.
51. The laser handpiece as set forth in example 50, wherein the second plurality of light transmitters is further configured to transmit light from the distal end to the proximal end.
52. The laser handpiece as set forth in example 51, further comprising a beam splitter coupled to at least part of the second plurality of light transmitters.
53. The laser handpiece as set forth in example 50, further comprising a microprocessor coupled to the light sensor to interpret the light received from the second plurality of light transmitters at the proximal end.
54. The laser handpiece as set forth in example 50, wherein the first plurality of light transmitters is capable of transmitting light comprising at least one of visible light, infrared light, blue light, and laser light.
55. The laser handpiece as set forth in example 54, wherein the elongate body comprises:
   a rigid portion; and
   at least one substantially flexible portion.
56. The laser handpiece as set forth in example 55, wherein at least one substantially flexible portion comprises a jointed section.
57. The laser handpiece as set forth in example 56, wherein the jointed section assumes in a neutral position an angle of about 15 to 20 degrees with respect to an axis of the rigid portion of the elongate body.
58. The laser handpiece as set forth in example 50, wherein:
   the first plurality of light transmitters comprises a first plurality of optical fibers; and
   the second plurality of light transmitters comprises a second plurality of optical fibers.
59. The laser handpiece as set forth in example 58, wherein the first plurality of light transmitters comprises at least one light altering element capable of influencing light transmitted to the distal end.
60. The laser handpiece as set forth in example 59, wherein the at least one light altering element comprises at least one optical filter.
61. The laser handpiece as set forth in example 50, further comprising:
   a third plurality of light transmitters extending from the proximal end to the distal end, the third plurality of light transmitters being configured to receive light from the distal end and to direct the light to the proximal end; and
   a camera coupled to receive light from at least part of the third plurality of light transmitters.
62. The laser handpiece as set forth in example 61, wherein light transmitted from the distal end to the proximal end by the second plurality of light transmitters is substantially the same as light received by, and directed to the proximal end by, the third plurality of light transmitters.
63. The laser handpiece as set forth in example 50, wherein the light sensor includes a camera coupled to receive light from at least part of the second plurality of light transmitters.
64. The laser handpiece as set forth in example 50, wherein the laser handpiece further comprises a camera that is coupled to receive light from the second plurality of light transmitters.
65. The laser handpiece as set forth in example 50, further comprising a beam splitter coupled to one or more of (a) at least part of the first plurality of light transmitters and (b) at least part of the second plurality of light transmitters.
66. The laser handpiece as set forth in example 65, wherein the light sensor is coupled to receive light from part of the second plurality of light transmitters.
67. The laser handpiece as set forth in example 65, further comprising a camera coupled to receive light from at least part of the second plurality of light transmitters.
68. The laser handpiece as set forth in example 65, further comprising:
   a third plurality of light transmitters extending from the proximal end to the distal end, the third plurality of light transmitters being capable of receiving light from the distal end and of directing the light to the proximal end; and
   a camera coupled to receive light from at least part of the third plurality of light transmitters.

## Claims

1. An electromagnetic energy device comprising:
an electromagnetic energy base unit (30);
a connector (40) that connects to the electromagnetic energy base unit (30);
a conduit (35) that connects to the connector (40); and
an electromagnetic energy handpiece (20), that connects to the conduit (35), wherein the electromagnetic energy handpiece (20) is capable of receiving electromagnetic energy, and one or more of illumination light and excitation light having a propagation path that envelops at least a part of a propagation path of the electromagnetic energy within the electromagnetic energy handpiece (20),
**characterized in that**
the handpiece is furthermore capable of receiving fluid from the electromagnetic energy base unit (30),
wherein the electromagnetic energy handpiece (20) comprises a handpiece tip (45), the handpiece tip (45) comprising:
a plurality of mirrors (420, 425);
a fiber tip (55, 400);
a plurality of tip waveguides (430), capable of receiving one or more of electromagnetic energy, illumination light, and excitation light, and directing the one or
more of electromagnetic energy, illumination light, and excitation light to a target surface; and
a housing (22) having disposed therein the plurality of tip waveguides (430), the fiber tip (55, 400), and a plurality of fluid outputs (41), wherein
the plurality of tip waveguides (430) and the plurality of fluid outputs (415) are circularly disposed around the fiber tip (55, 400).

2. The electromagnetic energy device as set forth in claim 1, wherein the fluid comprises spray water, spray air, and cooling air.

3. The electromagnetic energy device as set forth in claim 1 or 2, wherein the plurality of tip waveguides (430) comprises nine tip waveguides (430) separated by about forty degrees; and
the plurality of fluid outputs (415) comprises three fluid outputs (415) separated by about one hundred twenty degrees, wherein especially:
the nine tip waveguides (430) are disposed with respect to a reference at zero, forty, eighty, one hundred twenty, one hundred sixty, two hundred, two hundred forty, two hundred eighty, and three hundred twenty degrees; and
the three fluid outputs (415) are disposed with respect to the reference at one hundred, two hundred twenty, and three hundred forty degrees.

4. The electromagnetic energy device as set forth in one of the preceding claims, wherein the handpiece tip (45) comprises a housing (22) having disposed therein transparent material capable of transmitting light.

5. The electromagnetic energy device as set forth in claim 4, wherein the transparent material comprises one of transparent plastic, sapphire, and quartz.

6. The electromagnetic energy device as set forth in claim 1, wherein the handpiece (20) further comprises an elongate
portion (22) coupled to receive the concentrated electromagnetic energy and additional electromagnetic energy from the connector (40), wherein the handpiece tip (45) is formed as an extension of the elongate portion (22), the handpiece tip (45) being capable of directing electromagnetic energy to the target surface; and the handpiece (20) comprises
a first mirror (425) disposed in a general vicinity between opposing ends of the handpiece (20) and the elongate portion (22) and being capable of directing the additional electromagnetic energy through at least part of the handpiece tip (45) and to the target surface; and
a second mirror (420) eclipsing at least a part of the first mirror (425), relative to a direction of propagation of additional electromagnetic energy to the first mirror (425), and being capable of directing the concentrated electromagnetic energy through at least part of the handpiece tip (45) and to the target surface.

7. The electromagnetic energy device as set forth in claim 6, wherein:
the first mirror (425) is capable of receiving additional electromagnetic energy from one or more conduits and directing the additional electromagnetic energy to one or more of the tip waveguides (430) of the handpiece tip (45), the tip waveguides (430) being configured to direct the additional electromagnetic energy toward the target surface; and
the second mirror (420) is capable of receiving concentrated electromagnetic energy from the electromagnetic energy conduit (35) and directing concentrated the electromagnetic energy to the fiber tip (55) of the handpiece tip (45), the fiber tip (55) being configured to direct the concentrated
electromagnetic energy toward the target surface, wherein:
the elongate portion (22) is coupled to receive the spray water from the connector (40);
and
the elongate portion (22) comprises a spray water line coupled to receive the spray water and to route the spray water through at least part of the elongate portion (22) to the handpiece tip (45), and/or
wherein the additional electromagnetic energy comprises illumination light and excitation light that are both routed through at least part of the elongate portion (22) to the handpiece tip (45).

8. The electromagnetic energy device as set forth in the second alternative of claim 7, wherein the illumination light and the excitation light are routed through at least part of the elongate portion (22) by way of an illumination fiber (405) and an excitation fiber (405), or wherein the elongate handpiece (20) is further adapted to receive spray water or air from the connector (40) wherein preferably the air is supplied to the elongate handpiece (20) by way of a spray air line and a cooling air line.

9. The electromagnetic energy device as set forth in claim 6, wherein the elongate portion (22) comprises:
an electromagnetic energy fiber capable of receiving concentrated electromagnetic energy from the connector (40);
an illumination fiber (405) capable of receiving a portion of the additional electromagnetic energy as illumination light;
an excitation fiber (405) capable of receiving another portion of the additional electromagnetic energy as excitation light; and
a feedback fiber (410) capable of receiving feedback light from the handpiece tip (45).

10. The electromagnetic energy device as set forth in claim 9, the first mirror (425) being disposed within the handpiece tip (45) and being capable of directing the additional light as illumination light into a plurality of the tip waveguides, the second mirror (420) being disposed within the handpiece tip (45) and being capable of directing the concentrated electromagnetic energy into the fiber tip (55), and the tip waveguides being capable of receiving illumination and excitation light from the first mirror (425) and directing the illumination light to the target surface and receiving reflected light from the target surface and directing the reflected light to the first mirror (420).

11. The electromagnetic energy device as set forth in claim 10, further comprising at least one feedback fiber (410) disposed within the handpiece tip (45) and within the elongate portion (22), wherein the feedback fiber (410) is capable of receiving reflected light from the first mirror (425) and directing the feedback light to the connector (40), wherein especially the electromagnetic energy base unit (30) comprises a photo detector capable of receiving the feedback light from the connector (40) and providing a feedback display according to one of an error condition and a potential error condition in optical components of the electromagnetic energy handpiece (20).

12. The electromagnetic energy device as set forth in claim 7, further comprising:
first tubing disposed within the elongate portion (22) and the handpiece tip (45), the first tubing being capable of receiving air from the connector (40); and
second tubing disposed within the elongate portion (22) and the handpiece tip (45), the second tubing being capable of receiving water from the connector (40), wherein the electromagnetic energy handpiece (20) further comprises one or more mixing chambers, each mixing chamber having two inputs (413, 414) and a fluid output (415), the two inputs comprising:
a first input (413) capable of receiving air from first tubing; and
a second input (414) capable of receiving water from second tubing, wherein the air and water are mixed within the mixing chamber, and a mixture of air and water is expelled from the fluid output (415), wherein preferably the one or more mixing chambers comprise three mixing chambers.

13. A method of analyzing feedback light from a medical electromagnetic energy handpiece, thereby monitoring integrity of optical components, the method comprising:
providing an electromagnetic energy device according to one of the preceding claims;
receiving feedback light into the medical electromagnetic energy handpiece;
generating an electrical signal according to the feedback light; and
providing an error indication when the electrical signal exceeds a predetermined threshold.

14. The method as set forth in claim 13, wherein the providing of an error indication comprises generating a display on a monitor of an electromagnetic energy base unit.

## Patentansprüche

1. Vorrichtung für elektromagnetische Energie, Folgendes umfassend:
eine Basiseinheit (30) für elektromagnetische Energie;
einen Verbinder (40), der mit der Basiseinheit (30) für elektromagnetische Energie verbindbar ist;
einen Leiter (35), der mit dem Verbinder (40) verbindbar ist; und
ein Handstück (20) für elektromagnetische Energie, das mit dem Leiter (35) verbindbar ist, wobei das Handstück (20) für elektromagnetische Energie in der Lage ist, elektromagnetische Energie aufzunehmen, und wobei Beleuchtungslicht und/oder Anregungslicht einen Ausbreitungsweg aufweisen, der innerhalb des Handstücks (20) für elektromagnetische Energie wenigstens einen Teil eines Ausbreitungswegs der elektromagnetischen Energie umgibt,
**dadurch gekennzeichnet, dass**
das Handstück ferner in der Lage ist, Fluid aus der Basiseinheit (30) für elektromagnetische Energie aufzunehmen,
wobei das Handstück (20) für elektromagnetische Energie eine Handstückspitze (45) umfasst, wobei die Handstückspitze (45) Folgendes umfasst:
mehrere Spiegel (420, 425);
eine Faserspitze (55, 400);
mehrere Spitzenwellenleiter (430), die in der Lage sind, elektromagnetische Energie, Beleuchtungslicht und/oder Anregungslicht aufzunehmen und die elektromagnetische Energie, das Beleuchtungslicht und/oder das Anregungslicht zu einer Zieloberfläche zu leiten; und
ein Gehäuse (22), in dem die mehreren Spitzenwellenleiter (430), die Faserspitze (55, 400) und mehrere Fluidausgänge (41) angeordnet sind, wobei
die mehreren Spitzenwellenleiter (430) und die mehreren Fluidausgänge (415) kreisförmig um die Faserspitze (55, 400) herum angeordnet sind.

2. Vorrichtung für elektromagnetische Energie nach Anspruch 1, wobei das Fluid Sprühwasser, Sprühluft und Kühlungsluft umfasst.

3. Vorrichtung für elektromagnetische Energie nach Anspruch 1 oder 2, wobei die mehreren Spitzenwellenleiter (430) neun Spitzenwellenleiter (430) umfassen, die um etwa vierzig Grad voneinander beabstandet sind; und
wobei die mehreren Fluidausgänge (415) drei Fluidausgänge (415) umfassen, die um etwa einhundertzwanzig Grad voneinander beabstandet sind, wobei insbesondere:
die neun Spitzenwellenleiter (430) mit Bezug auf einen Bezugspunkt bei null, vierzig, achtzig, einhundertzwanzig, einhundert sechzig, zweihundert, zweihundert vierzig, zweihundertachtzig und dreihundertzwanzig Grad angeordnet sind; und
die drei Fluidausgänge (415) mit Bezug auf den Bezugspunkt bei einhundert, zweihundertzwanzig und dreihundertvierzig Grad angeordnet sind.

4. Vorrichtung für elektromagnetische Energie nach einem der vorhergehenden Ansprüche, wobei die Handstückspitze (45) ein Gehäuse (22) umfasst, in dem transparentes Material angeordnet ist, das Licht übertragen kann.

5. Vorrichtung für elektromagnetische Energie nach Anspruch 4, wobei das transparente Material transparenten Kunststoff, Saphir oder Quarz umfasst.

6. Vorrichtung für elektromagnetische Energie nach Anspruch 1,
wobei das Handstück (20) ferner einen länglichen Abschnitt (22) umfasst, der gekoppelt ist, um die konzentrierte elektromagnetische Energie und zusätzliche elektromagnetische Energie von dem Verbinder (40) aufzunehmen, wobei die Handstückspitze (45) als eine Verlängerung des länglichen Abschnitts (22) ausgebildet ist, wobei die Handstückspitze (45) elektromagnetische Energie zu der Zieloberfläche leiten kann; und das Handstück (20) Folgendes umfasst:
einen ersten Spiegel (425), angeordnet in einer allgemeinen Nähe zwischen einander gegenüberliegenden Enden des Handstücks (20) und des länglichen Abschnitts (22) und in der Lage, die zusätzliche elektromagnetische Energie durch wenigstens einen Teil der Handstückspitze (45) und zu der Zieloberfläche zu leiten; und
einen zweiten Spiegel (420), der wenigstens einen Teil des ersten Spiegels (425) verdeckt, bezogen auf eine Ausbreitungsrichtung einer zusätzlichen elektromagnetischen Energie hin zu dem ersten Spiegel (425), und in der Lage die konzentrierte elektromagnetische Energie durch wenigstens einen Teil der Handstückspitze (45) und zu der Zieloberfläche zu leiten.

7. Vorrichtung für elektromagnetische Energie nach Anspruch 6, wobei:
der erste Spiegel (425) in der Lage ist, zusätzliche elektromagnetische Energie aus einem oder mehreren Leitern aufzunehmen und die zusätzliche elektromagnetische Energie in einen oder mehrere der Spitzenwellenleiter (430) der Handstückspitze (45) zu leiten, wobei die Spitzenwellenleiter (430) konfiguriert sind, die zusätzliche elektromagnetische Energie zu der Zieloberfläche zu leiten; und
der zweite Spiegel (420) in der Lage ist, konzentrierte elektromagnetische Energie von dem Leiter (35) für elektromagnetische Energie aufzunehmen und die konzentrierte elektromagnetische Energie zu der Faserspitze (55) der Handstückspitze (45) zu leiten, wobei die Faserspitze (55) konfiguriert ist, die konzentrierte elektromagnetische Energie zu der Zieloberfläche zu leiten, wobei:
der längliche Abschnitt (22) gekoppelt ist, um Sprühwasser von dem Verbinder (40) aufzunehmen; und
der längliche Abschnitt (22) eine Sprühwasserleitung umfasst, die gekoppelt ist, um das Sprühwasser aufzunehmen und das Sprühwasser durch wenigstens einen Teil des länglichen Abschnitts (22) zu der Handstückspitze (45) zu führen, und/oder wobei die zusätzliche elektromagnetische Energie Beleuchtungslicht und Anregungslicht umfasst, die beide durch wenigstens einen Teil des länglichen Abschnitts (22) zu der Handstückspitze (45) geführt werden.

8. Vorrichtung für elektromagnetische Energie nach der zweiten Alternative in Anspruch 7, wobei das Beleuchtungslicht und das Anregungslicht mittels einer Beleuchtungsfaser (405) und einer Anregungsfaser (405) durch wenigstens einen Teil des länglichen Abschnitts (22) geführt werden, oder wobei das längliche Handstück (20) ferner angepasst ist, Sprühwasser oder Luft von dem Verbinder (40) aufzunehmen, wobei vorzugsweise die Luft mittels einer Sprühluftleitung und einer Kühlungsluftleitung an das längliche Handstück (20) bereitgestellt wird.

9. Vorrichtung für elektromagnetische Energie nach Anspruch 6, wobei der längliche Abschnitt (22) Folgendes umfasst:
eine Faser für elektromagnetische Energie, die in der Lage ist, konzentrierte elektromagnetische Energie von dem Verbinder (40) aufzunehmen;
eine Beleuchtungsfaser (405), die in der Lage ist, einen Teil der zusätzlichen elektromagnetischen Energie als Beleuchtungslicht aufzunehmen;
eine Anregungsfaser (405), die in der Lage ist, einen weiteren Teil der zusätzlichen elektromagnetischen Energie als Anregungslicht aufzunehmen; und
eine Feedbackfaser (410), die in der Lage ist, Feedbacklicht von der Handstückspitze (45) aufzunehmen.

10. Vorrichtung für elektromagnetische Energie nach Anspruch 9, wobei der erste Spiegel (425) in der Handstückspitze (45) angeordnet ist und in der Lage ist, das zusätzliche Licht als Beleuchtungslicht in mehrere der Spitzenwellenleiter zu leiten, wobei der zweite Spiegel (420) in der Handstückspitze (45) angeordnet ist und in der Lage ist, die konzentrierte elektromagnetische Energie in die Faserspitze (55) zu leiten, und die Spitzenwellenleiter in der Lage sind, Beleuchtungs- und Anregungslicht von dem ersten Spiegel (425) aufzunehmen und das Beleuchtungslicht zu der Zieloberfläche zu leiten und von der Zieloberfläche zurückgeworfenes Licht aufzunehmen und das zurückgeworfene Licht zu dem ersten Spiegel (425) zu leiten.

11. Vorrichtung für elektromagnetische Energie nach Anspruch 10, ferner wenigstens eine innerhalb der Handstückspitze (45) und innerhalb des länglichen Abschnitts (22) angeordnete Feedbackfaser (410) umfassend, wobei die Feedbackfaser (410) in der Lage ist, zurückgeworfenes Licht von dem ersten Spiegel (425) aufzunehmen und das Feedbacklicht zu dem Verbinder zu leiten (40), wobei insbesondere die Basiseinheit (30) für elektromagnetische Energie einen Lichtsensor umfasst, der in der Lage ist, das Feedbacklicht von dem Verbinder (40) aufzunehmen und eine Feedbackanzeige auf der Grundlage eines Fehlerzustands oder eines potenziellen Fehlerzustands in optischen Komponenten des Handstücks (20) für elektromagnetische Energie bereitzustellen.

12. Vorrichtung für elektromagnetische Energie nach Anspruch 7, ferner Folgendes umfassend:
ein erstes Rohr, angeordnet innerhalb des länglichen Abschnitts (22) und der Handstückspitze (45), wobei das erste Rohr in der Lage ist, Luft aus dem Verbinder (40) aufzunehmen; und
ein zweites Rohr, angeordnet innerhalb des länglichen Abschnitts (22) und der Handstückspitze (45), wobei das zweite Rohr in der Lage ist, Wasser von dem Verbinder (40) aufzunehmen, wobei das Handstück (20) für elektromagnetische Energie ferner eine oder mehrere Mischkammern umfasst, wobei jede Mischkammer zwei Eingänge (413, 414) und einen Fluidausgang (415) aufweist, wobei die zwei Eingänge Folgendes umfassen:
einen ersten Eingang (413), der in der Lage ist, Luft aus dem ersten Rohr aufzunehmen; und
einen zweiten Eingang (414), der in der Lage ist, Wasser aus dem zweiten Rohr aufzunehmen, wobei die Luft und das Wasser in der Mischkammer vermischt werden und ein Gemisch aus Luft und Wasser aus dem Fluidausgang (415) ausgegeben wird, wobei vorzugsweise die eine oder die mehreren Mischkammern drei Mischkammern umfassen.

13. Verfahren zum Analysieren von Feedbacklicht von einem medizinischen Handstück für elektromagnetische Energie und dadurch Überwachen der Integrität von optischen Komponenten, wobei das Verfahren Folgendes umfasst:
Bereitstellen einer Vorrichtung für elektromagnetische Energie nach einem der vorhergehenden Ansprüche;
Aufnehmen von Feedbacklicht in das medizinische Handstück für elektromagnetische Energie;
Erzeugen eines elektrischen Signals auf der Grundlage des Feedbacklichts; und
Bereitstellen einer Fehlerangabe, wenn das elektrische Signal einen vorgegebenen Schwellenwert übersteigt.

14. Verfahren nach Anspruch 13, wobei das Bereitstellen einer Fehlerangabe das Erzeugen einer Anzeige auf einem Monitor einer Basiseinheit für elektromagnetische Energie umfasst.

## Revendications

1. Dispositif à énergie électromagnétique comportant :
une unité de base à énergie électromagnétique (30),
un connecteur (40) qui est raccordé à l'unité de base à énergie électromagnétique (30),
un conduit (35) qui est raccordé au connecteur (40), et
une pièce à main à énergie électromagnétique (20), qui est raccordée au conduit (35), dans lequel la pièce à main à énergie électromagnétique (20) peut recevoir de l'énergie électromagnétique, et une ou plusieurs lumières parmi une lumière d'éclairage et une lumière d'excitation ayant un trajet de propagation qui enveloppe au moins une partie d'un trajet de propagation de l'énergie électromagnétique à l'intérieur de la pièce à main à énergie électromagnétique (20),
**caractérisé en ce que**
la pièce à main peut en outre recevoir du fluide provenant de l'unité de base à énergie électromagnétique (30),
dans lequel la pièce à main à énergie électromagnétique (20) comporte un embout de pièce à main (45), l'embout de pièce à main (45) comportant :
une pluralité de miroirs (420, 425),
un embout de fibre (55, 400),
une pluralité de guides d'ondes d'embout (430), pouvant recevoir un ou plusieurs éléments parmi de l'énergie électromagnétique, de la lumière d'éclairage et de la lumière d'excitation, et diriger l'élément ou les éléments parmi l'énergie électromagnétique, la lumière d'éclairage et la lumière d'excitation vers une surface cible, et
un boîtier (22) ayant disposé dans celui-ci la pluralité de guides d'ondes d'embout (430), l'embout de fibre (55, 400) et une pluralité de sorties de fluide (41),
dans lequel la pluralité de guides d'ondes d'embout (430) et la pluralité de sorties de fluide (415) sont disposées circulairement autour de l'embout de fibre (55, 400).

2. Dispositif à énergie électromagnétique selon la revendication 1, dans lequel le fluide comporte de l'eau pulvérisée, de l'air pulvérisé et de l'air de refroidissement

3. Dispositif à énergie électromagnétique selon la revendication 1 ou 2, dans lequel la pluralité de guides d'ondes d'embout (430) comporte neuf guides d'ondes d'embout (430) séparés d'environ quarante degrés, et
la pluralité de sorties de fluide (415) comporte trois sorties de fluide (415) séparées d'environ cent vingt degrés, dans lequel notamment :
les neuf guides d'ondes d'embout (430) sont disposés par rapport à une référence à zéro, quarante, quatre-vingt, cent vingt, cent soixante, deux cents, deux cent quarante, deux cent quatre-vingt et trois cent vingt degrés, et
les trois sorties de fluide (415) sont disposées par rapport à la référence à cent, deux cent vingt et trois cent quarante degrés.

4. Dispositif à énergie électromagnétique selon l'une des revendications précédentes, dans lequel l'embout de pièce à main (45) comporte un boîtier (22) ayant disposé dans celui-ci un matériau transparent pouvant transmettre de la lumière.

5. Dispositif à énergie électromagnétique selon la revendication 4, dans lequel le matériau transparent comporte un matériau parmi une matière plastique transparente, du saphir et du quartz.

6. Dispositif à énergie électromagnétique selon la revendication 1,
dans lequel la pièce à main (20) comporte en outre une portion allongée (22) couplée de manière à recevoir l'énergie électromagnétique concentrée et de l'énergie électromagnétique supplémentaire provenant du connecteur (40), dans lequel l'embout de pièce à main (45) est formé comme une extension de la portion allongée (22), l'embout de pièce à main (45) pouvant diriger l'énergie électromagnétique vers la surface cible, et la pièce à main (20) comporte :
un premier miroir (425) disposé à proximité entre des extrémités opposées de la pièce à main (20) et la portion allongée (22) et pouvant diriger l'énergie électromagnétique supplémentaire à travers au moins une partie de l'embout de pièce à main (45) et vers la surface cible, et
un second miroir (420) éclipsant au moins une partie du premier miroir (425), par rapport à une direction de propagation de l'énergie électromagnétique supplémentaire jusqu'au premier miroir (425), et pouvant diriger l'énergie électromagnétique concentrée à travers au moins une partie de l'embout de pièce à main (45) et vers la surface cible.

7. Dispositif à énergie électromagnétique selon la revendication 6, dans lequel :
le premier miroir (425) peut recevoir de l'énergie électromagnétique supplémentaire provenant d'un ou plusieurs conduits et peut diriger l'énergie électromagnétique supplémentaire vers un ou plusieurs des guides d'ondes d'embout (430) de l'embout de pièce à main (45), les guides d'ondes d'embout (430) étant configurés pour diriger l'énergie électromagnétique supplémentaire vers la surface cible, et
le second miroir (420) peut recevoir de l'énergie électromagnétique concentrée provenant du conduit d'énergie électromagnétique (35) et diriger l'énergie électromagnétique concentrée vers l'embout de fibre (55) de l'embout de pièce à main (45), l'embout de fibre (55) étant configuré pour diriger l'énergie électromagnétique concentrée vers la surface cible, dans lequel :
la portion allongée (22) est couplée de manière à recevoir l'eau pulvérisée provenant du connecteur (40), et
la portion allongée (22) comporte une ligne d'eau pulvérisée couplée de manière à recevoir l'eau pulvérisée et acheminer l'eau pulvérisée à travers au moins une partie de la portion allongée (22) jusqu'à l'embout de pièce à main (45), et/ou
dans lequel l'énergie électromagnétique supplémentaire comporte de la lumière d'éclairage et de la lumière d'excitation qui sont toutes deux transmises à travers au moins une partie de la portion allongée (22) jusqu'à l'embout de pièce à main (45).

8. Dispositif à énergie électromagnétique selon la seconde variante de la revendication 7, dans lequel la lumière d'éclairage et la lumière d'excitation sont acheminées à travers au moins une partie de la portion allongée (22) au moyen d'une fibre d'éclairage (405) et d'une fibre d'excitation (405), ou dans lequel la pièce à main allongée (20) est en outre adaptée pour recevoir de l'eau ou de l'air pulvérisé provenant du connecteur (40), dans lequel l'air est de préférence fourni à la pièce à main allongée (20) au moyen d'une ligne d'air de pulvérisation et d'une ligne d'air de refroidissement.

9. Dispositif à énergie électromagnétique selon la revendication 6, dans lequel la portion allongée (22) comporte :
une fibre d'énergie électromagnétique pouvant recevoir de l'énergie électromagnétique concentrée provenant du connecteur (40),
une fibre d'éclairage (405) pouvant recevoir une portion de l'énergie électromagnétique supplémentaire sous forme de lumière d'éclairage,
une fibre d'excitation (405) pouvant recevoir une autre portion de l'énergie électromagnétique supplémentaire sous forme de lumière d'excitation, et
une fibre de retour (410) pouvant recevoir de la lumière de retour provenant de l'embout de pièce à main (45).

10. Dispositif à énergie électromagnétique selon la revendication 9, le premier miroir (425) étant disposé à l'intérieur de l'embout de pièce à main (45) et pouvant diriger la lumière supplémentaire sous forme de lumière d'éclairage dans une pluralité des guides d'ondes d'embout, le second miroir (420) étant disposé à l'intérieur de l'embout de pièce à main (45) et pouvant diriger l'énergie électromagnétique concentrée dans l'embout de fibre (55), et les guides d'ondes d'embout pouvant recevoir la lumière d'éclairage et d'excitation provenant du premier miroir (425) et pouvant diriger la lumière d'éclairage vers la surface cible et recevoir la lumière réfléchie par la surface cible et diriger la lumière réfléchie vers le premier miroir (425).

11. Dispositif à énergie électromagnétique selon la revendication 10, comportant en outre au moins une fibre de retour (410) disposée à l'intérieur de l'embout de pièce à main (45) et à l'intérieur de la portion allongée (22), dans lequel la fibre de retour (410) peut recevoir la lumière réfléchie par le premier miroir (425) et peut diriger la lumière de retour vers le connecteur (40), dans lequel l'unité de base à énergie électromagnétique (30) comporte en particulier un photodétecteur pouvant recevoir la lumière de retour provenant du connecteur (40) et pouvant fournir un affichage de retour en fonction d'un élément parmi une condition d'erreur et une condition d'erreur potentielle dans des composants optiques de la pièce à main à énergie électromagnétique (20).

12. Dispositif à énergie électromagnétique selon la revendication 7, comportant en outre :
un premier tube disposé à l'intérieur de la portion allongée (22) et de l'embout de pièce à main (45), le premier tube pouvant recevoir de l'air provenant du connecteur (40), et
un second tube disposé à l'intérieur de la portion allongée (22) et de l'embout de pièce à main (45), le second tube pouvant recevoir de l'eau provenant du connecteur (40), dans lequel la pièce à main à énergie électromagnétique (20) comporte en outre une ou plusieurs chambres de mélange, chaque chambre de mélange ayant deux entrées (413, 414) et une sortie de fluide (415), les deux entrées comportant :
une première entrée (413) pouvant recevoir de l'air provenant du premier tube, et
une seconde entrée (414) pouvant recevoir de l'eau provenant du second tube, dans lequel l'air et l'eau sont mélangés à l'intérieur de la chambre de mélange, et un mélange d'air et d'eau est expulsé de la sortie de fluide (415), dans lequel la ou les chambres de mélange comportent de préférence trois chambres de mélange.

13. Procédé d'analyse d'une lumière de retour provenant d'une pièce à main médicale à énergie électromagnétique, en surveillant ainsi l'intégrité de composants optiques, le procédé comportant les étapes consistant à :
fournir un dispositif à énergie électromagnétique selon l'une des revendications précédentes,
recevoir de la lumière de retour dans la pièce à main médicale à énergie électromagnétique,
générer un signal électrique en fonction de la lumière de retour, et
fournir une indication d'erreur lorsque le signal électrique dépasse un seuil prédéterminé.

14. Procédé selon la revendication 13, dans lequel la fourniture d'une indication d'erreur comporte la génération d'un affichage sur un moniteur d'une unité de base à énergie électromagnétique.
